# EUROPÄISCHE PATENTSCHRIFT

(11) **EP 1 738 745 B1**
(45) Veröffentlichungstag und Bekanntmachung des Hinweises auf die Patenterteilung: **04.09.2013**
(21) Anmeldenummer: 06116033.9
(22) Anmeldetag: 26.06.2006
(51) Int. Cl.: A61K 8/49, A61Q 17/02, A01N 47/16, A01N 25/02, A01N 25/34

(54) **Emulsion mit Insekten-Repellentien**
Emulsion with insect repellents
Emulsion insectifuge

(30) Priorität: 27.06.2005 DE 102005030017
(43) Veröffentlichungstag der Anmeldung: 03.01.2007
(73) Patentinhaber: Beiersdorf AG, 20253 Hamburg (DE)
(72) Erfinder: Schulz, Jens, 22869 Schenefeld (DE); Von der Fecht, Stephanie, 22869 Schenefeld (DE); Nielsen, Jens, 25448 Henstedt-Ulzburg (DE); Kröpke, Rainer, 22869 Schenefeld (DE)

(56) Entgegenhaltungen:
- WO-A-2005/063016
- WO-A2-02/43656
- WO-A2-03/020232

## Beschreibung

Die vorliegende Erfindung betrifft eine kosmetische Emulsion mit einer wässrigen inneren Phase und einer lipophilen äußeren Phase, enthaltend 1-Piperidincarboxylsäure 2-(2-hydrooyethyl)-1-methylpropylester (INN: Icaridin).

Insektenschutzmittel (Insektenvertreibungsmittel, Insektenabwehrmittel, Repellentien, Repellents) sind Präparate, die zur Abwehr und/oder Vertreibung von Insekten, aber auch Zecken und Milben äußerlich angewendet werden und verhindern sollen, dass diese auf der Haut aktiv werden. Insektenabwehrmittel sollen die Haut vor Belästigung durch Blut saugende oder beißende Insekten und andere Parasiten und/oder Lästlinge schützen, indem sie diese abwehren, bevor sie sich auf der Haut niederlassen. Die Mittel wirken dementsprechend nicht als Kontaktgifte, sondern nur als Abwehrmittel, da sie die Tiere nicht töten, sondern lediglich vertreiben.

Demgemäß sind im Sinne der vorliegenden Erfindung unter dem Begriff "Insektenschutzmittel" nicht nur solche Präparate zu verstehen, die gegen Insekten (insbesondere Mücken) wirksam sind. Vielmehr gilt das nachstehend Gesagte selbstverständlich auch für solche Präparate, die andere Blut saugende oder beißende Parasiten und/oder Lästlinge (z.B. Spinnen, Flöhe, Milben) abwehren oder vertreiben, auch wenn dies im Einzelfall nicht erwähnt sein mag.

Schon seit Urzeiten werden die Menschen von stechenden oder beißenden Insekten oder anderen Parasiten geplagt. Dementsprechend alt ist das Bedürfnis der Menschheit nach Insektenabwehrmitteln. Eine schon seit der Frühgeschichte bekannte Methode, lästigen oder schädlichen Insekten ihren Aufenthalt in der Nähe des Menschen unattraktiv oder unangenehm zu machen, ist das Anzünden von Feuern mit aromatisch oder streng riechenden Kräutern oder Hölzern und starker Rauchentwicklung. Auch die Behandlung der Haut mit stark riechenden Substanzen zur Abwehr von Insekten ist bereits seit der Antike bekannt. Um die letzte Jahrhundertwende war eine Reihe natürlicher etherischer Öle als Insektenabwehrmittel im Gebrauch, so beispielsweise Anisöl, Bergamottöl, Birkenholzteer, Campher, Citronellöl, Eucalyptusöl, Geraniumöl, Kiefernöle, Kokosnußöl, Lavendelöl, Muskatnußöl, Nelkenöl, Orangenblütenöl, Pfefferminzöl, Poleiöle (Pennyroyalöl), Pyrethrum, Thymianöl und Zimtöl.

Wegen ihrer trotz intensiven Geruchs unzureichenden Wirksamkeit und ihrer zum Teil mangelnden Verträglichkeit in höheren Konzentrationen wurden diese Stoffe in heutigen Insektenabwehrmitteln weitgehend durch besser wirksame synthetische Substanzen verdrängt. Es handelt sich dabei überwiegend um hoch siedende Flüssigkeiten oder niedrig schmelzende bzw. sublimierende kristalline Stoffe, die bei Raumtemperatur langsam verdampfen. Die meisten Repellent-Wirkstoffe gehören den Stoffklassen der Amide, Alkohole, Ester und Ether an.

Ein moderner Repellent-Wirkstoff ist beispielsweise der 1-Piperidincarboxylsäure 2-(2-hydroxyethyl)-1-methylpropylester (INN: Icaridin, CAS-Nummer: 119515-38-7, Elincs-Nummer: 423-210-8), der die folgende Struktur 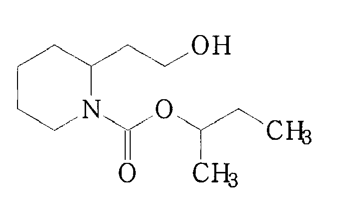 und einen bei 20 °C einen Dampfdruck von 3,4 10⁻⁴ hPa aufweist. 1-Piperidincarboxylsäure 2-(2-hydroxyethyl)-1-methylpropylester gilt als besonders wirksamer Repellent-Wirkstoff.

Zubereitungen mit 1-Piperidincarboxylsäure 2-(2-hydroxyethyl)-1-methylpropylester als Wirkstoff werden nach dem Stand der Technik in Form von Lösungen, Emulsionen, Dispersionen hergestellt, bei welchen der lipophile Wirkstoff von einer wässrigen Phase umgeben ist (z.B. O/W-Emulsion). Durch die Umhüllung des Wirkstoffs mit der wässrigen Phase wird dessen Freisetzung gebremst und die Zubereitung selbst ist bei der Anwendung auf der Haut länger wirksam gegen Mücken, Insekten, etc.

Nachteilig am Stande der Technik ist jedoch der Sachverhalt, das diese Zubereitungen aufgrund ihrer wässrigen äußeren Phase nicht besonders wasserfest sind und leicht von der Haut abgespült werden können. Ferner lassen sich in diesen Zubereitungen keine oxidationsempfindlichen, wasserlöslichen Zusatzstoffe einarbeiten.

Es war daher die Aufgabe der vorliegenden Erfindung, die Mängel des Standes der Technik zu beseitigen und neue Zubereitungsformen für den kosmetischen Einsatz von 1-Piperidincarboxylsäure 2-(2-hydroxyethyl)-1-methylpropylester auf der Haut zu entwickeln, bei denen sich der Wirkstoff zusammen mit wasserlöslichen, oxidationsempfindlichen Zusatzstoffen kombinieren lässt und die eine erhöhte Wasserfestigkeit auf der Haut aufweisen.

Überraschend gelöst werden die Aufgaben durch eine kosmetische Emulsion mit einer wässrigen inneren Phase und einer lipophilen äußeren Phase, enthaltend 1-Piperidincarboxylsäure 2-(2-hydroxyethyl)-1-methylpropylester (INN: Icaridin) mit einer wässrigen inneren Phase und einer lipophilen äußeren Phase in Form einer W/S-Emulsion, dadurch gekennzeichnet, dass die Emulsion einen oder mehrere W/S-Emulgatoren gewählt aus der Gruppe der Verbindungen ethoxylierten-proxylierten alkylmodifizierten Dimethicone und Dimethicone Copolyole enthält.

Überraschend war insbesondere der Umstand, dass, obwohl der Repellent-Wirkstoff in der äußeren, lipophilen Phase angereichert ist, bei der Anwendung auf der Haut gleichmäßig über einen Zeitraum von mehreren Stunden an die Umwelt abgegeben wird und somit eine lang anhaltende Repellent-Wirkung entfaltet.

Überraschend war nicht zuletzt, das bei den erfindungsgemäßen Zubereitungen die Klebrigkeit der Zubereitung auf der Haut im Vergleich zu Produkten des Stand der Technik (mit gleichem Repellent-Gehalt) deutlich reduziert ist.

Erfindungsgemäß ist ferner ein Packmittel aus Polyethylen, Polypropylen oder Polyethylenterephthalat enthaltend eine erfindungsgemäße kosmetische Emulsion mit einer wässrigen inneren Phase und einer lipophilen äußeren Phase, enthaltend 1-Piperidincarboxylsäure 2-(2-hydroxyethyl)-1-methylpropylester (INN: Icaridin).

Erfindungsgemäß ist nicht zuletzt ein Tuch oder Pflaster getränkt mit einer erfindungsgemäßen kosmetischen Emulsion mit einer wässrigen inneren Phase und einer lipophilen äußeren Phase, enthaltend 1-Piperidincarboxylsäure 2-(2-hydroxyethyl)-1-methylpropylester (INN: Icaridin).

Zwar kennt der Stand der Technik die WO 02/43656, die WO 03/020232 und die WO 2005/063016, doch konnten diese Schriften nicht den Weg zur vorliegenden Erfindung weisen.

Es ist erfindungsgemäß von Vorteil, wenn die erfindungsgemäße Emulsion in den erfindungsgemäßen Ausführungsformen 1-Piperidincarboxylsäure 2-(2-hydroxyethyl)-1-methylpropylester (INN: Icaridin) in einer Konzentration von 0,1 bis 40 Gewichts-% und bevorzugt in einer Konzentration von 10 bis 20 Gewichts-%, jeweils bezogen auf das Gesamtgewicht der Zubereitung enthalten.

Es ist vorteilhaft im Sinne der vorliegenden Erfindung, wenn die Emulsion Emulgatoren in einer Gesamtkonzentration von 0,5 bis 12,5 Gewichts-% und bevorzugt in einer Konzentration von 2,5 bis 8,0 Gewichts-%, jeweils bezogen auf das Gesamtgewicht der Zubereitung, enthält.

Es ist erfindungsgemäß einen oder mehrere W/S-Emulgatoren aus der Gruppe der Verbindungen ethoxylierten-proxylierten alkylmodifizierten Dimethicone und Dimethicone Copolyole zu wählen.

Dabei ist der Einsatz der W/S-Emulgatoren Dimethiconcopolyol und Cetyl PEG/PPG-10/1 Dimethicone erfindungsgemäß bevorzugt.

Liegt in der vorliegenden Erfindung die Emulsion, in Form einer W/S-Emulsion vor, so ist es erfindungsgemäß vorteilhaft, wenn sie eine oder mehrere lipophile Verbindungen gewählt aus der Gruppe der folgenden Verbindungen enthält: Dimethicone und Cyclomethicone.

Liegt in den erfindungsgemäßen Ausführungsformen der vorliegenden Erfindung die Emulsion in Form einer W/S-Emulsion vor, so ist es erfindungsgemäß vorteilhaft, wenn die Lipide einen Schmelzpunkt von 25-40°C aufweisen.

Die Wasserphase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder isopropanol, Diole oder Polyole niedriger C-Zahl sowie deren Ether, vorzugsweise Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, Polymere, Schaumstabilisatoren, Elektrolyte, Selbstbräuner sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Neben den erfindungsgemäßen Repellent-Wirkstoffen kann die erfindungsgemäße Zubereitung weitere kosmetische Wirk- und Pflegestoffe enthalten, beispielsweise die nach der Kosmetikverordnung zugelassenen UV-Lichtschutzfilter, und Konservierungsmittel bzw. Konservierungshelfer. Derartige Wirkstoffe können erfindungsgemäß vorteilhaft in Konzentrationen von 0,01 bis 30 Gew-%, bezogen auf das Gesamtgewicht der Zubereitung in dieser enthalten sein.

Als weitere Pflegestoffe können insbesondere Niacinamid, Panthenol, Aloe vera, Hammamelis-Extrakt, Polidocanol, Vitamin E, Vitamin E-Derivate, Vitamin A, Vitamin A-Derivate, Vitamin C, Vitamin C-Derivate, Coenzym Q10, Kreatin, Taurin, Alphaglycosylrutin und/oder in Konzentrationen von 0,1 bis 30 Gewichts-%, bezogen auf das Gesamtgewicht der Zubereitung eingesetzt werden.

Erfindungsgemäß besonders bevorzugt enthält die erfindungsgemäße Zubereitung als weitere Bestandteile Alpha-Hydroxysäuren und/oder deren Salze. Dabei werden erfindungsgemäß bevorzugt Milchsäure/Lactate oder Zitronensäure/Citrate in einer Konzentration von 0,01 bis 5 Gewichts-%, bezogen auf das Gesamtgewicht der Zubereitung eingesetzt.

Die Zubereitung kann erfindungsgemäß vorteilhaft in Form einer dünnflüssigen, sprühfähigen wässrigen, festen oder wässrig-alkoholischen Lösung, in Form eines Gels, als Salbe, Creme oder Lotion (ggf. sprühbar) vorliegen.

Das erfindungsgemäße Packmittel liegt erfindungsgemäß vorteilhaft in Form einer Tube oder einer Kunststoff-Flasche vor. Dabei sind Kunststoff-Flaschen erfindungsgemäß bevorzugt. Eine erfindungsgemäß besonders bevorzugte Ausführungsform stellen Kunststoff-Flaschen mit aufgesetztem Pumpspender dar, mit welchem die erfindungsgemäße Zubereitung aus dem Packmittel gefördert und ggf. versprüht werden kann.

Es ist erfindungsgemäß bevorzugt, wenn das erfindungsgemäße Packmittel (als Flasche) aus Polyethylen (PE) gebildet wird. Erfindungsgemäß besonders bevorzugt ist dabei der Einsatz von "high density polyethylene"(HDPE nach DIN 7728, TI. 1, 01/1988).

Erfindungsgemäß ist insbesondere die Verwendung der erfindungsgemäßen Zubereitung zur Prophylaxe von Mückenstichen und Zeckenbissen.

Die nachfolgenden Beispiele sollen die vorliegende Erfindung verdeutlichen ohne sie einzuschränken. Die Angaben beziehen sich stets auf Gewichts-%, sofern nicht andere Angaben gemacht werden.

### W/O-Emulsionen

| | **1** | **2** | **3** | **4** | **5** |
|---|---|---|---|---|---|
| Triglycerindiisostearat | 1,0 | 0,5 | 0,25 | 2,0 | 3,0 |
| Diglycerindipolyhydroxystearat | 1,0 | 1,5 | 1,75 | 3,0 | 2,0 |
| Paraffinöl | 12,5 | 10,0 | 8,0 | 5,0 | 11,5 |
| Vaseline | 8,0 | 6,0 | 5,0 | 12,0 | 2,5 |
| hydrierte Kokosglyceride | 2,0 | 1,0 | 2,5 | 5,0 | 0,25 |
| Decyloleat | 0,5 | 0,75 | 1,0 | 2,0 | 0,25 |
| Octyldodecanol | 0,5 | 1,0 | 0,75 | 3,0 | 0,25 |
| Aluminiumstearat | 0,4 | 0,3 | 0,6 | 1,0 | 0,05 |
| Dicaprylylcarbonat | 0,1 | 0,05 | 0,15 | 0,5 | 1,0 |
| hydriertes Rizinusöl | 0,5 | 0,75 | 1,0 | 2,5 | 5,0 |
| Icaridin | 7,5 | 12,5 | 25 | 5 | 20 |
| Magnesiumsulfat | 0,5 | 0,6 | 0,5 | 0,7 | 1,0 |
| Glycerin | 3,0 | 5,0 | 10,0 | 15,0 | 1,5 |
| Zitronensäure | 0,2 | 0,1 | 0,2 | 0,3 | 1,0 |
| Natriumcitrat | 0,2 | 0.05 | 0.4 | 0.3 | 2,0 |
| Parfum | q,s, | q,s, | q,s, | q,s, | q,s, |
| Ethanol | 2,0 | --- | 5,0 | --- | --- |
| Capryl-/Caprinsäuretriglycerid | 2,0 | 2,5 | 3,0 | 5,0 | 0,5 |
| Kaliumsorbat | 0,04 | 0,15 | 0,05 | 0,03 | 0,4 |
| Benzylalkohol | 0,3 | 0,4 | 0,25 | 0,15 | --- |
| Talkum | --- | --- | 0,5 | 1,0 | 0,1 |
| Wasser | ad 100 | ad 100 | ad 100 | ad 100 | ad 100 |

### W/O-Emulsionen

| | **6** | **7** | **8** | **9** | **10** |
|---|---|---|---|---|---|
| PEG-30 Dipolyhydroxystearat | --- | 0,5 | 0,25 | --- | 3,0 |
| Lanolin Alcohol | 1,0 | 1,5 | 1,75 | 3,0 | -- |
| Paraffinöl | 12,5 | 10,0 | 8,0 | 5,0 | 10,5 |
| Vaseline | 8,0 | 6,0 | 5,0 | 12,0 | 2,5 |
| hydrierte Kokosglyceride | 2,0 | 1,0 | 2,5 | 5,0 | 0,25 |
| Hydriertes Polyisobuten | 0,5 | 0,75 | 1,0 | 2,0 | 0,25 |
| Octyldodecanol | 0,5 | 1,0 | 0,75 | 3,0 | 0,25 |
| Aluminiumstearat | 0,4 | 0,3 | 0,6 | 1,0 | 0,05 |
| Dicaprylylcarbonat | 0,1 | 0,05 | 0,15 | 0,5 | 1,0 |
| hydriertes Rizinusöl | 0,5 | 0,75 | 1,0 | 2,5 | 5,0 |
| Microcrystalline Cellulose | 0,5 | 1,0 | 0,75 | 0,25 | 0,1 |
| Magnesiumsulfat | 0,5 | 0,6 | 0,5 | 0,7 | 1,0 |
| Glycerin | 3,0 | 5,0 | 10,0 | 15,0 | 1,5 |
| Zitronensäure | 0,2 | 0,1 | 0,2 | 0,3 | 1,0 |
| Parfum | q,s, | q,s, | q,s, | q,s, | q,s, |
| 1,3 Butylenglykol | 2,0 | --- | 5,0 | --- | --- |
| Capryl-/Caprinsäuretriglycerid | 2,0 | 2,5 | 3,0 | 5,0 | 0,5 |
| Icaridin | 5,0 | 15,0 | 7,5 | 30 | 17,5 |
| Kaliumsorbat | 0,3 | 0,4 | 0,25 | 0,15 | --- |
| Talkum | --- | --- | 0,05 | --- | 0,1 |
| Wasser | ad 100 | ad 100 | ad 100 | ad 100 | ad 100 |

### W/S-Emulsion

| | **11** | **12** | **13** | **14** | **15** |
|---|---|---|---|---|---|
| Cetyl PEG/PPG-10/1 Dimethicone | 1,0 | --- | -- | 3,0 | 5,0 |
| Cylomethicon + PEG/PPG-18/18 Dimethicon (90:10) | 10,0 | 12,5 | 25 | -- | -- |
| Cyclomethicon | 12,5 | 15 | 22,0 | 20,0 | 15,5 |
| Dimethicon | 5,0 | 13,0 | 5,0 | 12,0 | 15,0 |
| hydriertes Polyisobuten | 0,5 | 0,75 | 1,0 | 2,0 | 0,25 |
| Octyldodecanol | 0,5 | 1,0 | 0,75 | 3,0 | 0,25 |
| Panthenol | 0,5 | 1,0 | 0,75 | 0,25 | 0,1 |
| Natriumchlorid | 2,0 | 0,6 | 2,5 | 0,7 | 1,0 |
| Glycerin | 3,0 | 5,0 | 10,0 | 15,0 | 1,5 |
| Zitronensäure | 0,2 | 0,1 | 0,2 | 0,3 | 1,0 |
| Natriumcitrat | 1,0 | 0,1 | 0,4 | 0,9 | 2,5 |
| Parfum | q,s, | q,s, | q,s, | q,s, | q,s, |
| Kaliumsorbat | 0,4 | 0,1 | 0,05 | 0,3 | 0,4 |
| Microcrystalline Cellulose | 1,0 | 0,1 | 0,5 | 0,25 | 0,1 |
| Icaridin | 2,5 | 12,5 | 10 | 17,5 | 25 |
| Benzylalkohol | --- | --- | 0,05 | --- | 0,1 |
| modifizierte Stärke | --- | 2,5 | --- | 0,15 | -- |
| Wasser | ad 100 | ad 100 | ad 100 | ad 100 | ad 100 |

### W/S-Emulsionen

| | **16** | **17** | **18** | **19** | **20** |
|---|---|---|---|---|---|
| Cetyl PEG/PPG-10/1 Dimethicone | 1,0 | --- | -- | 3,0 | 5,0 |
| Cylomethicon + PEG/PPG-18/18 Dimethicon (90:10) | 10,0 | 12,5 | 25 | -- | -- |
| Cyclomethicon | 12,5 | 15 | 8,0 | 20,0 | 17,5 |
| Dimethicon | 5,0 | 13,0 | 5,0 | 12,0 | 15,0 |
| hydriertes Polyisobuten | 0,5 | 0,75 | 1,0 | 2,0 | 0,25 |
| Octyldodecanol | 0,5 | 1,0 | 0,75 | 3,0 | 0,25 |
| Panthenol | 0,5 | 1,0 | 0,75 | 0,25 | 0,1 |
| Natriumchlorid | 2,0 | 0,6 | 2,5 | 0,7 | 1,0 |
| Glycerin | 3,0 | 5,0 | 10,0 | 15,0 | 1,5 |
| Milchsäure | 0,2 | 0,1 | 0,2 | --- | --- |
| Natriumlactat | 0,2 | 1,0 | 0.05 | --- | --- |
| Parfum | q,s, | q,s, | q,s, | q,s, | q,s, |
| Microcrystalline Cellulose | 1,0 | 0,1 | 1,5 | 2,5 | 0,1 |
| Kaliumsorbat | 0,3 | 0,4 | 0,25 | 0,15 | --- |
| Stearyldimethicon | 0,5 | --- | 0,7 | --- | --- |
| Icaridin | 12,5 | 22,5 | 30,0 | 15,0 | 11,0 |
| modifizierte Stärke | --- | 2,5 | --- | 0,15 | -- |
| Wasser | ad 100 | ad 100 | ad 100 | ad 100 | ad 100 |

### W/O-Emulsionen

| | **21** | **22** | **23** | **24** | **25** |
|---|---|---|---|---|---|
| PEG-22 Dodecyl Glycol Copolymer | 5,0 | 1,5 | 0,25 | --- | 8,0 |
| PEG-45 Dodecyl Glycol Polymer | 1,0 | 1,5 | 1,75 | 3,0 | -- |
| Paraffinöl | 12,5 | 10,0 | 8,0 | 5,0 | 17,5 |
| Isopropylstearat | 8,0 | 6,0 | 5,0 | 12,0 | 2,5 |
| hydrierte Kokosglyceride | 2,0 | 1,0 | 2,5 | 5,0 | 0,25 |
| Nachtkerzenöl | 0,5 | 0,75 | 1,0 | 2,0 | 0,25 |
| Octyldodecanol | 0,5 | 1,0 | 0,75 | 3,0 | 0,25 |
| Aluminiumstearat | 0,4 | 0,3 | 0,6 | 1,0 | 0,05 |
| Dicaprylylcarbonat | 0,1 | 0,05 | 0,15 | 0,5 | 1,0 |
| hydriertes Rizinusöl | 0,5 | 0,75 | 1,0 | 2,5 | 5,0 |
| Microcrystalline Cellulose | 0,5 | 1,0 | 0,75 | 0,25 | 0,1 |
| Magnesiumsulfat | 0,5 | 0,6 | 0,5 | 0,7 | 1,0 |
| Glycerin | 3,0 | 5,0 | 10,0 | 15,0 | 1,5 |
| Natriumcitrat | 0,2 | 0,1 | --- | --- | --- |
| Zitronensäure | 0,2 | 0,1 | --- | --- | --- |
| Parfum | q,s, | q,s, | q,s, | q,s, | q,s, |
| 1,3 Butylenglykol | 2,0 | --- | 5,0 | --- | --- |
| Capryl-/Caprinsäuretriglycerid | 2,0 | 2,5 | 3,0 | 5,0 | 0,5 |
| Kaliumsorbat | 0,4 | 0,15 | 0,05 | 0,3 | 0,4 |
| Talkum | 0,3 | 0,4 | 0,25 | 0,15 | --- |
| Icaridin | 5 | 10 | 15 | 20 | 25 |
| Wasser | ad 100 | ad 100 | ad 100 | ad 100 | ad 100 |

### W/O-Emulsionen

| | **26** | **27** | **28** | **29** | **30** |
|---|---|---|---|---|---|
| Polyglyceryl-2-dipolyhydroxystearat | 3,0 | --- | 0,25 | --- | 3,0 |
| Polyglyceryl-3 diisostearate | 1,0 | 3,5 | 1,75 | 2,5 | -- |
| PEG-40 sorbitanisostearate | --- | 2,5 | 0,5 | 3,5 | 3,0 |
| Paraffinöl | 12,5 | 10,0 | 8,0 | 5,0 | 11,5 |
| Isopropylstearat | 8,0 | 6,0 | 5,0 | 12,0 | 2,5 |
| hydrierte Kokosglyceride | 2,0 | 1,0 | 2,5 | 5,0 | 0,25 |
| Isopropylpalmitat | 0,5 | 1,0 | 0,75 | 3,0 | 0,25 |
| Dicaprylylcarbonat | 0,1 | 0,05 | 0,15 | 0,5 | 1,0 |
| hydriertes Rizinusöl | 0,5 | 0,75 | 1,0 | 2,5 | 5,0 |
| Microcrystalline Cellulose | 0,5 | 1,0 | 0,75 | 0,25 | 0,1 |
| Magnesiumsulfat | 0,5 | 0,6 | 0,5 | 0,7 | 1,0 |
| Glycerin | 3,0 | 5,0 | 10,0 | 15,0 | 1,5 |
| Zitronensäure | 0,2 | 0,1 | 0,1 | 0,3 | 1,0 |
| Natriumcitrat | 0,2 | 0,3 | 0,2 | 1,5 | 0.8 |
| Parfum | q,s, | q,s, | q,s, | q,s, | q,s, |
| Capryl-/Caprinsäuretriglycerid | 2,0 | 2,5 | 3,0 | 5,0 | 0,5 |
| Kaliumsorbat | 0,24 | 0,15 | 0,05 | 0,3 | 0,4 |
| Icaridin | 5 | 10 | 15 | 20 | 30 |
| Wasser | ad 100 | ad 100 | ad 100 | ad 100 | ad 100 |

## Patentansprüche

1. Kosmetische Emulsion enthaltend 1-Piperidincarboxylsäure 2-(2-hydroxyethyl)-1-methylpropylester (INN: Icaridin)
mit einer wässrigen inneren Phase und einer lipophilen äußeren Phase in Form einer W/S-Emulsion, **dadurch gekennzeichnet, dass** die Emulsion einen oder mehrere W/S-Emulgatoren gewählt aus der Gruppe der Verbindungen ethoxylierten-proxylierten alkylmodifizierten Dimethicone und Dimethicone Copolyole enthält.

2. Packmittel aus Polyethylen, Polypropylen oder Polyethylenterephthalat enthaltend eine Emulsion nach Anspruch 1.

3. Tuch oder Pflaster getränkt mit einer Emulsion nach Anspruch 1.

4. Emulsion, Packmittel, Tuch oder Pflaster nach einem der vorhergehenden Ansprüche,
**dadurch gekennzeichnet, dass** die Emulsion 1-Piperidincarboxylsäure 2-(2-hydroxyethyl)-1-methylpropylester (INN: Icaridin) in einer Konzentration von 0,1 bis 40
Gewichts-%, bezogen auf das Gesamtgewicht der Zubereitung enthält.

5. Emulsion, Packmittel, Tuch oder Pflaster nach einem der vorhergehenden Ansprüche, **dadurch gekennzeichnet, dass** die Emulsion Emulgatoren in einer Gesamtkonzentration von 0,5 bis 12,5 Gewichts-%, bezogen auf das Gesamtgewicht der Zubereitung, enthält.

6. W/S-Emulsion, Packmittel, Tuch oder Pflaster nach einem der vorhergehenden Ansprüche, **dadurch gekennzeichnet, dass** sie eine oder mehrere lipophile Verbindungen gewählt aus der Gruppe der folgenden Verbindungen enthält: Dimethicone und Cyclomethicone.

7. W/S-Emulsion, Packmittel, Tuch oder Pflaster nach einem der vorhergehenden Ansprüche, **dadurch gekennzeichnet, dass** sie eine oder mehrere Lipide mit einen Schmelzpunkt von 25-40°C aufweisen.

## Claims

1. Cosmetic emulsion comprising 2-(2-hydroxyethyl)-1-methylpropyl 1-piperidinecarboxylate (INN: Icaridin) with an aqueous internal phase and a lipophilic external phase in the form of a W/S emulsion, **characterized in that** the emulsion comprises one or more W/S emulsifiers selected from the group of the compounds ethoxylated-propoxylated alkyl-modified dimethicones and dimethicone copolyols.

2. Pack made of polyethylene, polypropylene or polyethylene terephthalate comprising an emulsion according to Claim 1.

3. Wipe or plaster impregnated with an emulsion according to Claim 1.

4. Emulsion, pack, wipe or plaster according to one of the preceding claims, **characterized in that** the emulsion comprises 2-(2-hydroxyethyl)-1-methylpropyl 1-piperidinecarboxylate (INN: Icaridin) in a concentration of from 0.1 to 40% by weight, based on the total weight of the preparation.

5. Emulsion, pack, wipe or plaster according to one of the preceding claims, **characterized in that** the emulsion comprises emulsifiers in a total concentration of from 0.5 to 12.5% by weight, based on the total weight of the preparation.

6. W/S emulsion, pack, wipe or plaster according to one of the preceding claims, **characterized in that** it comprises one or more lipophilic compounds selected from the group of the following compounds: dimethicones and cyclomethicones.

7. W/S emulsion, pack, wipe or plaster according to one of the preceding claims, **characterized in that** they have one or more lipids with a melting point of 25-40°C.

## Revendications

1. Emulsion cosmétique contenant de l'ester 2-(2-hydroxyéthyl)-1-méthylpropylique de l'acide 1-pipéridinecarboxylique (INN : Icaridin) présentant une phase interne aqueuse et une phase externe lipophile sous forme d'une émulsion E/S, **caractérisée en ce que** l'émulsion contient un ou plusieurs émulsifiants E/S choisis dans le groupe des composés de type diméthicone éthoxylé-prvpoxylé modifié par alkyle et diméthicone-copolyol.

2. Emballage en polyéthylène, en polypropylène ou en poly(téréphtalate d'éthylène) contenant une émulsion selon la revendication 1.

3. Tissu ou pansement imbibé d'une émulsion selon la revendication 1.

4. Emulsion, emballage, tissu ou pansement selon l'une quelconque des revendications précédentes, caractérisé(e) en ce que l'émulsion contient l'ester 2-(2-hydroxyéthyl)-1-méthylpropylique de l'acide 1-pipéridinecarboxylique (INN : Icaridin) en une concentration de 0,1 à 40% en poids, par rapport au poids total de la composition.

5. Emulsion, emballage, tissu ou pansement selon l'une quelconque des revendications précédentes, caractérisé(e) en ce que l'émulsion contient des émulsifiants en une concentration totale de 0,5 à 12,5% en poids, par rapport au poids total de la préparation.

6. Emulsion E/S, emballage, tissu ou pansement selon l'une quelconque des revendications précédentes, caractérisé(e) en ce qu'il/elle contient un ou plusieurs composés lipophiles choisis dans le groupe des composés suivants : diméthicones et cyclométhicones.

7. Emulsion E/S, emballage, tissu ou pansement selon l'une quelconque des revendications précédentes, caractérisé(e) en ce qu'il/elle présente un ou plusieurs lipides présentant un point de fusion de 25-40°C.
